# EUROPEAN PATENT SPECIFICATION

(11) **EP 2 926 817 B1**
(45) Date of publication and mention of the grant of the patent: **29.03.2017**
(21) Application number: 13859631.7
(22) Date of filing: 30.10.2013
(51) Int. Cl.: A61K 31/675, A61K 31/593, A61P 19/10, A61K 9/28, A61K 9/50, A61K 9/20

(54) **STABLE PHARMACEUTICAL COMPOSITION FOR TREATING OSTEOPOROSIS**
STABILE PHARMAZEUTISCHE ZUSAMMENSETZUNG ZUR BEHANDLUNG VON OSTEOPOROSE
COMPOSITION PHARMACEUTIQUE STABLE POUR LE TRAITEMENT DE L'OSTÉOPOROSE

(30) Priority: 03.12.2012 MX 2012014071
(43) Date of publication of application: 07.10.2015
(73) Proprietor: Landsteiner Scientific S.A. de C.V., 01900 Distrito Federal (MX)
(72) Inventor: GARCÍA MONDRAGÓN, María Lourdes, Estado de México (MX); REYES SANTIAGO, Alejandro, 52773 Huixquilucan Estado de México (MX)
(74) Representative: Covadonga Fernández-Vega Feijoo, Maria
(86) International application number: PCT/MX2013/000130
(87) International publication number: WO 2014/088385

(56) References cited:
- EP-A2- 2 478 909
- WO-A1-2010/090614
- MX-A- 2012 002 786
- US-A1- 2005 260 262
- US-A1- 2010 158 998
- DATABASE WPI Week200924, Derwent Publications Ltd., London, GB; AN 2009-B49998, XP003033308 & KR 10 0 844 256 B1 (KOLON PHARMACEUTICAL CO LTD) 07 July 2008
- DATABASE WPI Week201267, Derwent Publications Ltd., London, GB; AN 2012-B26208, XP003033309 & KR 2012 0 005 228 A (NAVIPHARM CO LTD) 16 January 2012

## Description

### FIELD OF THE INVENTION

This invention refers to stable compositions which consist of therapeutically effective doses of risedronate and cholecalciferol used to inhibit bone resorption, especially osteoporosis.

### BACKGROUND OF THE INVENTION

Bone resorption leads to a reduction in bone mass, causing diseases such as postmenopausal osteoporosis, osteoporosis in males, glucocorticoid-induced osteoporosis and Paget's disease (which involves the bone destruction and abnormal regeneration, causing deformity), among others. The appearance of osteoporosis increases with age and therefore there are greater complications associated with bone fracture, as well as higher medical costs.

The treatment of bone resorption and related bone diseases requires medications which use a bisphosphonate as an active ingredient, which inhibits bone resorption. The bisphosphonates used includes the salts of the alendronic, pamidronic, risedronic, ibandronic and zolendronic acids.

It has been reported that the administration of bisphosphonates is associated with hypocalcemia, since they inhibit bone resorption by osteoclasts, preventing calcium leakage from the bone, and consequently reducing calcium concentration in the blood. The reduction of calcium in the blood produces a homeostatic increase in the level of parathyroid hormone in the blood, which is dangerous for a patient receiving treatment for vitamin D deficiency, parathyroid dysfunction, poor liver function or Paget's disease, requiring the appropriate intake of calcium and vitamin D before and during the bisphosphonate treatment.

Given that a bisphosphonate treatment increases the calcium requirement, calcium absorption must be increased in order to achieve an effective osteoporosis treatment. Therefore, the level of vitamin D in the blood is an important factor.

Based on the foregoing, vitamin D intake is recommended during bisphosphonate treatment. The normal recommended dose is between 400 and 800 UI.

Vitamin D increases calcium absorption in the small intestine, and plays an important role in cellular differentiation in bone and in the generation of high quality bone matrix in the osteoblasts. It is important to maintain proper levels of vitamin D or its metabolites in the blood in order to improve bone metabolism as an effective treatment against osteoporosis.

The active form of vitamin D3 is chemically unstable in terms of temperature, oxygen and light, and therefore requires refrigeration, protection from light and the substitution of 02 by an inert gas. It is an insaponifiable heterolipid from the steroid group, and the active forms of Vitamin D3 is the 1α- hydroxycholecalciferol, 1α, 25 - dihydroxy-cholecalciferol or 1 α24- dyhydroxycholecalciferol, also known as calcitriol, which is converted in the kidneys from the 25-hydroxycholecalciferol or calcitriol in the circulating blood, which in turn is converted in the liver from vitamin D3. It functions to increase calcium and phosphorus absorption in the intestine, induce the formation of osteoclasts for bone resorption (increases the calcium concentration in the blood) and reduce the production of parathyroid hormone (PTH).

Risedronate is a pyridinyl bisphosphonate that binds to the bone hydroxyapatite and inhibits bone resorption by osteoclasts, increases bone mass and skeletal biomechanical resistance, depending on the dose. Its activity was confirmed by measuring biochemical markers of bone remodeling.

Risedronate absorption is relatively quick after administering an oral dose (T_{max.} ∼1 hour), and its speed is independent of the dose (single dose of 2.5 to 30 mg; multiple dose of 2.5 to 5 mg daily, and up to 50 mg weekly). It has an average oral bioavailability of 0.63%, and is reduced with food; it produces similar effects in both men and women. The mean volume of distribution at steady state equilibrium is 6.3 L/kg in humans. The plasmatic protein binding is 24%. There is no evidence of systemic metabolism of risedronate sodium. One half of the absorbed dose is excreted in the urine during the first 24 hours, and 85% of an injected dose is recovered in the urine after 28 days.

It is prescribed in the treatment of post-menopausal osteoporosis, osteoporosis in males, glucocorticoid-induced osteoporosis, and Paget's disease. Side effects include: known hypersensitivity to risedronate sodium or to any of its excipients, hypocalcemia and serious renal failure.

With regard to technique, various combinations have been described, including risedronate and vitamin D; the application MX/a2012/002786, PHARMACEUTICAL COMPOSITIONS INCLUDING HIGH DOSE BISPHOSPHONATE DERIVATIVES AND CHOLECALCIFEROL, presents a pharmaceutical composition for the prevention or treatment of osteoporosis that may be administered once a month; the pharmaceutical composition includes risedronic acid (or its salt) and cholecalciferol, in a high dose. The composition includes: (a) granules containing cholecalciferol adsorbed in microcrystalline cellulose, the quantity of cholecalciferol is 24,000 to 50,000 UI; and one or more excipients chosen from tocopheryl acetate, butylhydroxytoluene, and butylhydroxyanisole as a first stabilizing agent; and a binding agent in ethanol or an aqueous ethanol solution, mannitol as a second stabilizing agent, and risedronic acid or its salt.

The Mexican application PA/a/2004/004807, COMPOSITIONS AND METHODS TO INHIBIT BONE RESORPTION, describes the compositions and methods used to prevent, inhibit, reduce or treat conditions and diseases associated with abnormal bone resorption in mammals, such as osteoporosis; the modalities of the compositions of the invention include a pharmaceutically effective quantity of alendronate and vitamin D3 prepared for a once-a-week dose; the compositions and methods of the invention provide vitamin D nutrition during the treatment with bisphosphonate to facilitate normal bone formation and mineralization, while reducing to a minimum the complications associated with vitamin D deficiency or the potential thereof, such as hypocalcemia and osteomalacia; it also describes methods for manufacturing compositions of the invention herein, for measuring the stability and degradation of the compositions, and for measuring vitamin D levels in blood plasma.

The medication known as Actonel Plus Ca & D includes enteric coated capsules containing 35 mg of risedronate, and effervescent granules which contain 1 g of calcium carbonate and 880 UI of vitamin D3. The capsules include lactose monohydrate, microcrystalline cellulose, crospovidone A, magnesium stearate; the enteric coating includes: hypromellose, macrogol, hydroxypropylcellulose, silicon dioxide, titanium dioxide (E171), red and yellow iron oxide (E172); the effervescent granules include: anhydrous citric acid, malic acid, gluconolactone, maltodextrin, sodium cyclamate, sodium saccharin, sorbitol E420, Mannitol E421; it also includes other excipients such as dextrin, acacia, natural lemon oil, lemon scent, rice starch, potassium carbonate, tocopheryl, hydrogenated soybean oil, gelatin, sucrose, corn oil.

Actonel is prescribed in the treatment of postmenopausal osteoporosis to reduce the risk of vertebral fractures. It is only prescribed for patients whose assessment indicates that the quantity of calcium and vitamin D3 that is included will provide their adequate supplement.

The weekly unit of Actonel Plus Ca & D consists of 1 coated tablet with 35 mg resonate and 6 packets of calcium and vitamin D3. The adult recommended dose is one 35 mg tablet of risedronate on the first day, followed by 1 packet of calcium, vitamin D3 and one more each day for 5 days. This 7-day sequence is repeated each week. The absorption of risedronate sodium is affected by foods; , in order to ensure the proper absorption, patients must take the tablet before breakfast: at least 30 minutes prior to the first food, medicine or drink (except water) of the day.

The tablet must be swallowed whole and may not be sucked or chewed. To facilitate the tablet's entry into the stomach, the patients must not lie down for 30 minutes after taking the tablet and must remain standing. The packet of calcium and vitamin D3 must be taken daily 6 days a week, starting the day after the risedronate tablet is taken. The contents of the packet are emptied into a glass of water, and must be stirred and drunk immediately.

The US patent numbers 7,473,684 dated January 6, 2009, and 8,093,228 dated January 10, 2012, both named: Formulation of Bisphosphonate, describe a bisphosphonate which includes risedronate for the treatment of osteoporosis, which is formulated with a quantity of anti-foaming agent that is effective in reducing the formation of foam in the stomach, reducing reflux and reduced esophageal irritation, achieving greater patient compliance.

The US patent application serial no. 2010/0048511 A1. COMPLEX FORMULATION TO PREVENT OR TREAT OSTEOPOROSIS INCLUDING THE SOLID DISPERSION OF VITAMIN D OR ITS DERIVATIVES AND BISPHOSPHONATE, describes a solid dispersion with vitamin D or a derivative thereof and a cyclodextrin, as well as a method for preparing the complex formulation. The complex formulation can maintain a constant therapeutic level of vitamin D or a derivative thereof via its improved stability, while improving patient compliance by minimizing discomfort and side effects when administered.

The publication WO2010090614 A1, PHARMACEUTICAL FORMULATION COMPRISING RISEDRONATE, CALCIUM CARBONATE AND VITAMIN D3 IN ONE COMBINED DOSE; the invention relates to pharmaceutical compositions which include the combination of the active ingredients in a single dose in order to increase patient compliance for the treatment of diseases and conditions associated with abnormal bone resorption. The combination includes a calcium salt, vitamin D and risedronate or pharmaceutically acceptable salt, derivative or hydrate thereof.

Therefore, it is plausible to have a composition that would coordinate the benefits of risedronate and vitamin D3, without involving the drawbacks of the compositions that are available so far.

### OBJECT OF THE INVENTION

The object of the present invention is to provide pharmaceutical compositions which increase the bioavailability of risedronate sodium, since it has very low absorption (0.63%). We propose increasing the bioavailability of risedronate to obtain a lower dose with the same effect.

A second object of the invention is to provide a shield for the Vitamin D3, by treating the Vitamin D3 powder to increase its stability through the use of suitable excipients and antioxidants containing an enteric coating.

Therefore, it provides a composition which can maintain Vitamin D3 in a stable form through a shielding process using excipients to form a co-processed excipient and to provide a transmembrane carrier, increasing the bioavailability (greater than 0.63%) of risedronate sodium and vitamin D.

### DESCRIPTION OF THE INVENTION

The pharmaceutical compositions of the present invention include a granulated core containing risedronate or a pharmaceutically acceptable salt thereof and at least one excipient, which acts as an antiresorptive agent on bone that is useful in treating osteoporosis; cholecalciferol, vitamin D3 with sprayed shielding on the risedronate granules, a first coating of the risedronate granules sprayed with the shielded cholecalciferol; pharmaceutically acceptable excipients for the preparation of a tablet made from risedronate granules sprayed with shielded cholecalciferol and coated; and an enteric coating on the tablet.

The composition comprises between 10 to 40% of the weight of the risedronic acid composition or its pharmaceutically acceptable derivatives, its salts, anfiolates and hydrates; between 0.001 to 0.05% of the weight of the composition of cholecalciferol, Vitamin D3; from 50 to 80% of the weight of the composition of at least one emulsifier, from 2 to 6% of the weight of the composition of at least one disintegrating agent, from 2 to 10% of the weight of the composition of at least one diluting agent, 0.1 to 2% by weight of the composition of at least one binding agent, from 0.2 to 0.5% of the weight of the composition of a first enteric coating, from 3 to 5% of the weight of the composition of a second enteric coating, from 0.25 to 5% of the weight of the composition of at least one lubricating agent and a sufficient amount of one or more solvents or solvent mixture as the dissolution medium.

Wherein the diluent is selected from the group including cellulose PH 102, microcrystalline cellulose PH 101, Ludipress, Prosolv 90; the disintegrating agent is selected from the group consisting of croscarmellose sodium, crospovidone, and sodium starch glycolate; the binder is selected from the group consisting of polyvinylpyrrolidone K 30, povidone PK 90, copovidone, hydroxypropyl methylcellulose, ethyl cellulose; the emulsifying agent is selected from the group consisting of Phosal 50 PG phosphatidylcholine in soy lecithin, propylene glycol, sunflower mono- and diglycerides and ascorbyl palmitate; the composition comprising an enteric coating is selected from NS Enteric and sodium alginate, Acryl-eze™, methacrylic acid copolymer, Sureteric®, polymethacrylates, triethylcitrate, triacetin, and polyethylene glycol; and solvents are selected from purified water, ethanol, propanol and mixtures thereof.

Preferably, the composition of the risedronate granule includes risedronate sodium, a disintegrating agent selected from the group consisting of croscarmellose sodium, microcrystalline cellulose and povidone K30.

The cholecalciferol shield consists of an emulsifier selected from the group comprised of Phosal, phosphatidylcholine in soy lecithin, propylene glycol, sunflower mono- and diglycerides and ascorbyl palmitate and ethanol as solvent. This solution is sprayed on the granulated risedronate sodium, and subsequently coated with a suspension of NS Enteric coating.

The tablet also includes one or more of the following excipients selected from croscarmellose sodium, microcrystalline cellulose, povidone K30, Phosal and NS Enteric agent and a lubricant, such as magnesium stearate.

The coating consists of a methacrylic acid copolymer (in Acryl-eze™), which gives the product gastro-resistant properties.

The following tables show examples of the compositions of this invention.

**Table 1.**

| **Component** | **Quantity** |
|---|---|
| Risedronate sodium | 35.00 mg |
| Vitamin D3 | 2000 UI |
| Microcrystalline cellulose PH 102 | 132.95 mg |
| Croscarmellose sodium | 9.00 mg |
| Polyvinylpyrrolidone K 30 | 10.00 mg |
| Phosal 50 PG | 1.00 mg |
| NS Enteric | 10.00 mg |
| Magnesium stearate | 2.00 mg |
| Ethanol | 48.62 mg |
| White Acryl-eze™ | 14.00 mg |
| Triethyl citrate | 1.40 mg |
| Opadry® FX | 0.60 mg |
| Purified water | 84.00 mg |
| **TOTAL** | **216.00 mg** |

**Table 2.**

| **Component** | **Quantity** |
|---|---|
| Risedronate sodium | 30.00 |
| Vitamin D3 | 2500 UI |
| Ludipress | 150 mg |
| Crospovidone | 12.0 mg |
| Hidroxymethylcellulose | 15.00 mg |
| Propylene glycol | 1.5 mg |
| NS Enteric | 10.00 mg |
| Magnesium stearate | 2.00 mg |
| Ethanol | 48.62 mg |
| Purified water | 84.00 mg |
| **TOTAL** | 220.5 mg |

**Table 3.**

| **Component** | **Quantity** |
|---|---|
| Risedronate sodium | 25.00 mg |
| Vitamin D3 | 2000 UI |
| Prosolv 90 | 150 mg |
| Croscarmellose sodium | 9.0 mg |
| Polyvinylpyrrolidone K 30 | 10.00 mg |
| Phosal 50 PG | 1.00 mg |
| Sodium alginate | 10.00 mg |
| Magnesium stearate | 2.00 mg |
| Ethanol | 48.62 mg |
| Methacrylate copolymer | 21.00 mg |
| Triacetin | 2.1 mg |
| Purified water | 84.00 mg |
| **TOTAL** | 207.05 mg |

The compositions presented above are prepared using the procedure described below for the composition in Table 1:

### PREPARATION OF THE GRANULE COMPOSITION:

Weigh, mix and granulate risedronate sodium, croscarmellose sodium, microcrystalline cellulose PH 102 and polyvinylpyrrolidone K 30 with purified water.
Sift using No. 14 mesh and dry in oven until less than 3.0% moisture is reached.

### PREPARATION OF VITAMIN D3 SHIELD

Dissolve vitamin D3 in Phosal 50 PG and subsequently dilute with ethyl alcohol, mix until it forms a clear, yellow solution.

Introduce the dry risedronate granules into a fluid bed basin, and permeate with the vitamin D3 spray solution at a temperature between 25° to 30° C.

### GRANULE ENTERIC COATING

Prepare a suspension of NS Enteric in purified water at 10% solids, stir until completely incorporated and sift using No. 50 mesh.

Spray the above suspension in the granules at a temperature of 25° to 35° C, dry the granules in a fluid bed and sift using No. 18 mesh.

### PREPARATION OF THE CORE

Sift using No. 20 mesh: microcrystalline cellulose PH 102, croscarmellose sodium and mix with the granules from step No. 6 for 5 minutes. Lubricate with magnesium stearate, previously sieved through No. 30 mesh, for 3 minutes.

Compress in 4 mm hexagonal press at 200 mg ± 5% of weight.

### ENTERIC COATING OF THE TABLET-CORE AND SHIELDED VITAMIN D3

Prepare a coating suspension with Acryl-eze™ and triethyl citrate in purified water at 20% solids.

Coat the tablets with the Acryl-eze™ suspension at a temperature of 30° to 35° C in a perforated drum.

Prepare the coating suspension with Opadry® FX in purified water to 6.5% solids.

Coat the tablets with the Opadry® FX suspension at a temperature of 38° to 43° C in a perforated drum.

Two stability tests of vitamin D3 and risedronate sodium were performed at different conditions by subjecting the tablets that were prepared according to the composition in Table 1. The conditions were as follows:
40°C/75% RH
30°C/65% RH
Cycled (40° C/75% RH-cooling).

The results of the tests that were performed are presented below:

### Tests:

**DISSOLUTION Acid Stage**

| | | **RISEDRONATE SODIUM** |
|---|---|---|
| | | **Acid Stage** |
| **Specification** | **Sample** | **% Dissolved** |
| | S1 | 0.56% |
| | S2 | 0.80% |
| | S3 | 0.95% |
| | S4 | 1.19% |
| No more than 10.0% | S5 | 0.71% |
| | S6 | 0.97% |
| | Mean | **0**.**86**% |
| | Maximum | 1.19% |
| | Minimum | 0.56% |
| | %RSD | 0.00% |

The product complies with the gastro-resistant stage, because it has an enteric coating to protect the mucous membranes while it passes through the gastrointestinal system.

**DISSOLUTION Buffer Stage**

| **pH=6.8** | |
|---|---|
| TIME (MIN) | Risedronate Sodium 35 mg |
| 5 | 94.08 |
| 10 | 100.93 |
| 15 | 100.84 |
| 20 | 100.94 |
| 30 | 100.12 |
| 45 | 100.48 |

It is evident that the risedronate sodium dissolves quickly in a medium with pH 6.8, obtaining more than 100% in the first 10 minutes.

### EVALUATION OF THE STABILITY OF VITAMIN D3

Two batches of the composition were subjected to different conditions to evaluate the stability of vitamin D3 and risedronate sodium. The conditions were the following: 30°C/65% RH, 40°C/75% RH and Cycled 40°C/75% RH-Cooling (2° - 8° C). The samples were submitted for 4 weeks and the following results were obtained.

**Results of Test 1**

| Conditions | | | |
|---|---|---|---|
| Week | 30°C/65% RH | 40°C/75% RH | Cycled |
| 0 | 100.00% | 100.00% | 100.00% |
| 1 | 102.02% | 98.34% | 98.69% |
| 2 | 99.20% | 98.12% | 97.90% |
| 4 | 96.39% | 96.03% | 96.09% |

**Results of Test 2**

| Conditions | | | | |
|---|---|---|---|---|
| Week | 30°C/65% RH | 40°C/75% RH | Cycled | |
| 0 | 100.00% | 100.00% | 100.00% | |
| 1 | 97.34% | 99.62% | 95.55% | |
| 2 | 96.93% | 97.80% | 96.31% | |
| 4 | 94.79% | 96.32% | 94.79% | |

**EVALUATION OF THE STABILITY OF RISEDRONATE SODIUM**

| | Test 1 | Test 2 |
|---|---|---|
| Initial | 102.55% | 100.99% |
| Final | 100.66% | 96.75% |

It is evident that vitamin D3 shows a slight decrease (5.96%) in the rating over the four weeks in which it was subjected to different conditions, and therefore we conclude that vitamin D3 achieved stabilization with the proposed manufacturing process.

On the other hand, it has been proven that the formulation meets the dissolution specifications in the acid stage and in the buffer stage.

The objective of the combination of sodium risedronate and vitamin D3 is to seek a synergetic relationship for the treatment of osteoporosis, the risedronate sodium inhibits bone resorption and osteoblast activity and bone mineralization by Vitamin D3 aids in calcium absorption; this is achieved in a single pharmaceutical form containing both active ingredients in a formulation which stabilizes vitamin D3, since it is chemically unstable in the environment. The risedronate sodium is a soluble but waterproof product; it has an added solubilizing agent that acts as a transmembrane carrier to increase the low bioavailability of the risedronate sodium, which will produce pharmacological effects in less time than the products currently available.

The tests performed on the product show that it was possible to stabilize the vitamin D3 without modifying the stability of the risedronate sodium, it meets pharmacopeial dissolution specifications for risedronate sodium, and a gastro-resistance test was performed to ensure that it does not come into contact with the mucous membranes, since that would cause severe irritation. In the buffer stage it was shown that the product is bioavailable in less than 30 minutes, to ensure that no problems will occur in the dissolution of the risedronate in the intestine, where the absorption will take place.

In conclusion, the pharmaceutical composition of risedronate sodium/vitamin D3 offers a comprehensive treatment for problems issuing from osteoporosis, as it is intended to improve the bioavailability of both active ingredients to produce a pharmacological effect in less time.

The vitamin D3 shield does increase its stability, since the blend of the emulsifier, antimicrobial and antioxidant agents contributes various functions which aid it in maintaining stability. According to the results that were obtained, the vitamin D3 rating is constant throughout the period in which it normally degrades.

The components of the pharmaceutical formulation also increase the bioavailability of risedronate sodium and the Phosal 50 PG contributes a phosphatidylcholine which is a component of cell membranes, and which therefore serves as a carrier to aid in the absorption of risedronate sodium.

## Claims

1. A stable pharmaceutical composition consisting of therapeutically effective doses of risedronate and cholecalciferol that is useful in the inhibition of bone resorption, **characterized by** a granular core containing risedronate or one of its pharmaceutically acceptable salts and at least one excipient; cholecalciferol, Vitamin D3 with shielding sprayed onto the risedronate granules, a first coating of the risedronate granules sprayed with shielded cholecalciferol; , pharmaceutically acceptable excipients for the preparation of a tablet from sprayed risedronate granules with shielded cholecalciferol and coatings, and an enteric coating on the tablet.

2. The composition according to claim 1, which is characterized because it comprises between 10 to 40% of the weight of the risedronic acid composition or its pharmaceutically acceptable derivatives, its salts, anfiolates and hydrates; between 0.001 to 0.05% of the weight of the composition of cholecalciferol, Vitamin D3; from 50 to 80% of the weight of the composition of at least one emulsifier, from 2 to 6% of the weight of the composition of at least one disintegrating agent, from 2 to 10% of the weight of the composition of at least one diluting agent, 0.1 to 2% by weight of the composition of at least one binding agent, from 0.2 to 0.5% of the weight of the composition of a first enteric coating, from 3 to 5% of the weight of the composition of a second enteric coating, from 0.25 to 5% of the weight of the composition of at least one lubricating agent and a sufficient amount of one or more solvents or solvent mixture as the dissolution medium.

3. The composition according to claim 1, wherein the diluent is selected from the group including cellulose PH 102, microcrystalline cellulose PH 101, Ludipress, Prosolv 90; the disintegrating agent is selected from the group consisting of croscarmellose sodium, crospovidone, and sodium starch glycolate; the binder is selected from the group consisting of polyvinylpyrrolidone K 30, povidone PK 90, copovidone, hydroxypropyl methylcellulose, ethyl cellulose; the emulsifying agent is selected from the group consisting of Phosal 50 PG, phosphatidylcholine in soy lecithin, propylene glycol, sunflower mono- and diglycerides and ascorbyl palmitate; the composition comprising an enteric coating is selected from NS Enteric and sodium alginate, Acryl-eze™, methacrylic acid copolymer, Sureteric®, polymethacrylates, triethylcitrate, triacetin, and polyethylene glycol; and solvents are selected from purified water, ethanol, propanol and mixtures thereof.

4. The composition according to claim 1, which is characterized because the risedronate granule composition includes risedronate sodium, a disintegrating agent selected from the group consisting of croscarmellose sodium, microcrystalline cellulose and povidone K30.

5. The composition according to claim 1, which is characterized because the cholecalciferol shield consists of an emulsifier selected from the group comprised of Phosal, phosphatidylcholine in soy lecithin, propylene glycol, sunflower mono- and diglycerides and ascorbyl palmitate and ethanol as solvent.

6. The composition according to claim 1, which is characterized because the cholecalciferol solution is sprayed on the granulated risedronate sodium, and subsequently coated with a suspension of NS Enteric coating.

7. The composition according to claim 1, which is characterized because the tablet also includes one or more excipients selected from croscarmellose sodium, microcrystalline cellulose, povidone K30, Phosal and NS Enteric agent and a lubricant, such as magnesium stearate.

8. The composition according to claim 1, which is characterized because the coating consists of a methacrylic acid copolymer.

9. A process for the preparation of the composition according to claim 1, characterized because it includes the following stages:
a) PREPARE THE GRANULE COMPOSITION
b) PREPARE THE VITAMIN D3 SHIELD
c) COAT THE GRANULE WITH AN ENTERIC LAYER
d) FORM THE CORE OF THE TABLET; AND
e) COAT THE TABLET-CORE AND SHIELDED VITAMIN D3 WITH AN ENTERIC LAYER.

10. The process according to claim 9, which is characterized because the preparation of THE GRANULE COMPOSITION consists of the following steps:
a) Weigh, mix and granulate risedronate sodium, croscarmellose sodium, microcrystalline cellulose PH 102 and polyvinylpyrrolidone K 30 with purified water.
b) Sift using No. 14 mesh and dry in oven until less than 3.0% moisture is reached.

11. The process according to claim 9, which is characterized because the preparation of the VITAMIN D3 SHIELD consists of the following steps:
a) Dissolve vitamin D3 in Phosal 50 PG and subsequently dilute with ethyl alcohol, mix until it forms a clear, yellow solution.
b) Introduce the dry risedronate granules into a fluid bed basin, and permeate with the vitamin D3 spray solution at a temperature between 25° to 30° C.

12. The process according to claim 9, which is characterized because the preparation of the GRANULE ENTERIC COATING consists of the following steps:
a) Prepare a suspension of NS Enteric in purified water at 10% solids, stir until completely incorporated and sift using No. 50 mesh.
b) Atomize the above suspension in the granules at a temperature of 25° to 35° C, dry the granules in a fluid bed and sift using No. 18 mesh.

13. The process according to claim 9, which is characterized because the GRANULE CORE FORMATION consists of the following steps:
a) Sift using No. 20 mesh: microcrystalline cellulose PH 102, croscarmellose sodium and mix with the granules from step No. 6 for 5 minutes.
b) Lubricate with magnesium stearate, previously sieved through No. 30 mesh, for 3 minutes.
c) Compress in 4 mm hexagonal press at 200 mg ± 5% of weight.

14. The process according to claim 9, which is characterized because the preparation of the ENTERIC COATING FOR THE TABLET-CORE AND SHIELDED VITAMIN D3 consists of the following steps:
a) Prepare a coating suspension with Acryl-eze™ and triethyl citrate in purified water at 20% solids.
b) Coat the tablets with the Acryl-eze™ suspension at a temperature of 30° to 35° C in a perforated drum.
c) Prepare the coating suspension with Opadry® FX in purified water to 6.5% solids.
d) Coat the tablets with the Opadry® FX suspension at a temperature of 38° to 43° C in a perforated drum.

15. The composition according to claim 1, which is characterized because the cholecalciferol has a stability greater than 93% after 4 weeks at 30°C/65% RH, 40°C/75% RH, and Cycled 40°C/75% RH and RH-Cooling (2°C - 8°C).

16. The composition according to claim 1, which is characterized because the dissolution of risedronate was no more than 10.0 at acid pH and is 100% at physiological pH at 10 minutes.

## Patentansprüche

1. Stabile pharmazeutische Zusammensetzung bestehend aus therapeutisch wirksamen Dosen von Risedronat und Cholecalciferol, welche bei der Hemmung der Knochenresorption nützlich ist, **gekennzeichnet durch** einen granulösen Kern enthaltend Risedronat oder eine seiner pharmazeutisch akzeptablen Salze und mindestens einen Hilfsstoff; Cholecalciferol, Vitamin D3 mit einer auf die Risedronatgranulate gesprühte Abschirmung, eine erste Beschichtung der Risedronatgranulate gesprüht mit abgeschirmten Cholecalciferol; pharmazeutisch akzeptable Hilfsstoffe für die Zubereitung einer Tablette aus gesprühten Risedronatgranulaten mit abgeschirmten Cholecalciferol und Beschichtungen, und eine enterische Beschichtung auf der Tablette.

2. Zusammensetzung nach Anspruch 1, **dadurch gekennzeichnet, dass** sie zwischen 10 und 40 Gew.-% Risedronsäure-Zusammensetzung oder deren pharmazeutisch akzeptablen Derivate, deren Salze, Anfiolate und Hydrate; zwischen 0,001 und 0,05 Gew.-% der Zusammensetzung Cholecalciferol, Vitamin D3; von 50 bis 80 Gew.-% der Zusammensetzung mindestens eines Emulgators, von 2 bis 6 Gew.-% der Zusammensetzung mindestens eines Desintegrationsmittels, von 2 bis 10 Gew.-% der Zusammensetzung mindestens eines Verdünnungsmittel, 0,1 bis 2 Gew.-% der Zusammensetzung mindestens eines Bindemittels, von 0,2 bis 0,5 Gew.-% der Zusammensetzung einer ersten enterischen Beschichtung, von 3 bis 5 Gew.-% der Zusammensetzung einer zweiten enterischen Beschichtung, von 0,25 bis 5 Gew.-% der Zusammensetzung mindestens eines Schmiermittels und eine ausreichende Menge eines oder mehrerer Lösemittel oder Lösemittelmischung als Lösungsmittel umfasst.

3. Zusammensetzung nach Anspruch 1, wobei das Verdünnungsmittel aus der Gruppe beinhaltend Cellulose PH 102, mikrokristalline Cellulose PH 101, Ludipress, Prosolv 90 ausgewählt wird; das Desintegrationsmittel aus der Gruppe bestehend aus Croscarmellose-Natrium, Crospovidon und Natrium-Stärkeglykolat ausgewählt wird; das Bindemittel aus der Gruppe bestehend aus Polyvinylpyrrolidon K 30, Povidon PK 90, Copovidon, Hydroxypropylmethylcellulose, Ethylcellulose ausgewählt wird; das Emulgiermittel aus der Gruppe bestehend aus Phosal 50 PG, Phosphatidylcholin in Sojalecithin, Propylenglykol, Mono- und Diglyceriden aus der Sonnenblume und Ascorbylpalmitat ausgewählt wird; wobei die Zusammensetzung eine enterische Beschichtung ausgewählt aus NS Enteric und Natriumalginat, Acryl-eze™, Methacrylsäure-Copolymer, Sureteric®, Polymethacrylaten, Triethylcitrat, Triacetin und Polyethylenglykol umfasst; und die Lösemittel aus gereinigtem Wasser, Ethanol, Propanol und Mischungen derselben ausgewählt werden.

4. Zusammensetzung nach Anspruch 1, **dadurch gekennzeichnet, dass** die Risedronatgranulat-Zusammensetzung Natrium-Risedronat und ein Desintegrationsmittel, ausgewählt aus der Gruppe bestehend aus Croscarmellose-Natrium, mikrokristalliner Cellulose und Povidon K30, beinhaltet.

5. Zusammensetzung nach Anspruch 1, **dadurch gekennzeichnet, dass** die Cholecalciferolabschirmung aus einem Emulgator besteht, welcher aus der Gruppe ausgewählt wird, welche aus Phosal, Phosphatidylcholin in Sojalecithin, Propylenglykol, Mono- und Diglyceriden aus der Sonnenblume besteht und Ascorbylpalmitat und Ethanol als Lösemittel.

6. Zusammensetzung nach Anspruch 1, **dadurch gekennzeichnet, dass** die Cholecalciferol-Lösung auf dem granulierten Natrium-Risedronat aufgesprüht wird, und anschließend mit einer Suspension aus NS Enteric Beschichtung beschichtet wird.

7. Zusammensetzung nach Anspruch 1, **dadurch gekennzeichnet, dass** die Tablette auch einen oder mehrere Hilfsstoffe, welche aus Croscarmellose-Natrium, mikrokristalliner Cellulose, Povidon K30, Phosal und NS Enteric Mittel ausgewählt werden, und ein Schmiermittel, wie Magnesiumstearat beinhaltet.

8. Zusammensetzung nach Anspruch 1, **dadurch gekennzeichnet, dass** die Beschichtung aus einem Methacrylsäure-Copolymer besteht.

9. Verfahren für die Zubereitung der Zusammensetzung nach Anspruch 1, **dadurch gekennzeichnet, dass** es die folgenden Schritte beinhaltet:
a) das Zubereiten der Granulatzusammensetzung;
b) das Zubereiten der Vitamin D3-Abschirmung;
c) das Beschichten der Granulate mit einer enterischen Schicht;
d) das Bilden des Kerns der Tablette; und
e) das Beschichten des Tablettenkerns und des abgeschirmten Vitamins D3 mit einer enterischen Schicht.

10. Verfahren nach Anspruch 9, **dadurch gekennzeichnet, dass** die Zubereitung der Granulatzusammensetzung aus den folgenden Schritten besteht:
a) das Wiegen, Mischen und Granulieren von Natrium-Risedronat, Croscarmellose-Natrium, mikrokristalliner Cellulose PH 102 und Polyvinylpyrrolidon K 30 mit gereinigtem Wasser;
b) das Durchsieben unter Verwendung einer Nr. 14 Masche und das Trocknen in einem Ofen bis weniger als 3,0% Feuchtigkeit erreicht wird.

11. Verfahren nach Anspruch 9, **dadurch gekennzeichnet, dass** die Zubereitung der Vitamin D3-Abschirmung aus den folgenden Schritten besteht:
a) das Lösen des Vitamins D3 in Phosal 50 PG und das anschließende Verdünnen mit Ethylalkohol und das Mischen bis sich eine transparente, gelbe Lösung bildet;
b) das Einführen der trockenen Risedronatgranulate in einen Wirbelbettbehälter, und das Imprägnieren mit der Vitamin D3-Sprühlösung bei einer Temperatur zwischen 25º und 30ºC.

12. Verfahren nach Anspruch 9, **dadurch gekennzeichnet, dass** die Zubereitung der enterischen Beschichtung der Granulate aus den folgenden Schritten besteht:
a) das Zubereiten einer Suspension aus NS Enteric in gereinigtem Wasser mit 10% Feststoffen, das Rühren bis zur vollständigen Einarbeitung und das Durchsieben unter Verwendung einer Nr. 50 Masche;
b) das Zerstäuben der oben erwähnten Suspension in die Granulate bei einer Temperatur von 25º bis 35ºC, das Trocknen der Granulate in einem Wirbelbett und das Durchsieben unter Verwendung einer Nr. 18 Masche.

13. Verfahren nach Anspruch 9, **dadurch gekennzeichnet, dass** das Bilden des Granulatkerns aus den folgenden Schritten besteht:
a) das Durchsieben unter Verwendung einer Nr. 20 Masche mikrokristalliner Cellulose PH 102, Croscarmellose-Natrium und das Mischen mit den Granulaten aus Schritt Nr. 6 während 5 Minuten;
b) das Schmieren mit Magnesiumstearat, welches zuvor durch eine Nr. 30 Masche, während 3 Minuten gesiebt wurde;
c) das Zusammenpressen in einer 4 mm hexagonalen Presse bei 200 mg ± 5% Gewicht.

14. Verfahren nach Anspruch 9, **dadurch gekennzeichnet, dass** die Zubereitung der enterischen Beschichtung für den Tablettenkern und das abgeschirmte Vitamin D3 aus den folgenden Schritten besteht:
a) das Zubereiten einer Beschichtungssuspension mit Acryl-eze™ und Triethylcitrat in gereinigtem Wasser bei 20% Feststoffen;
b) das Beschichten der Tabletten mit der Acryl-eze™-Suspension bei einer Temperatur von 30º bis 35ºC in einer perforierten Trommel;
c) das Zubereiten der Beschichtungssuspension mit Opadry® FX in gereinigtem Wasser bis 6,5% Feststoffen;
d) das Beschichten der Tabletten mit der Opadry® FX-Suspension bei einer Temperatur von 38º bis 43ºC in einer perforierten Trommel.

15. Zusammensetzung nach Anspruch 1, **dadurch gekennzeichnet, dass** das Cholecalciferol eine Stabilität grösser als 93% nach 4 Wochen bei 30ºC/65% rel. F., 40ºC/75% rel. F. aufweist, und Zyklierung bei 40ºC/75% rel. F. und Kühlung bei rel. F. (2ºC - 8C).

16. Zusammensetzung nach Anspruch 1, **dadurch gekennzeichnet, dass** die Lösung aus Risedronat nicht mehr als 10,0 bei saurem pH ist und 100% bei physiologischem pH nach 10 Minuten ist.

## Revendications

1. Composition pharmaceutique stable consistant en des doses thérapeutiquement effectives de risédronate et de cholécalciférol qui est utile dans l'inhibition de la résorption osseuse, **caractérisée par** un noyau nucléaire contenant du risédronate ou un de ses sels pharmaceutiquement acceptables et au moins un excipient; du cholécalciférol, de la vitamine D3 avec une protection pulvérisée sur les granulés de risédronate, une première couverture des granulés de risédronate pulvérisées avec du cholécalciférol protégé; des excipients pharmaceutiquement acceptables pour la préparation d'un comprimé à partir de granules de risédronate pulvérisés avec du cholécalciférol protégé et des recouvrements, et un recouvrement entérique sur le comprimé.

2. Composition selon la revendication 1, qui est **caractérisée en ce qu'**elle comprend entre 10 et 40% du poids de la composition d'acide risédronique ou ses dérivés, ses sels, ses anfiolates, et ses hydrates pharmaceutiquement acceptables; entre 0,001 et 0,05% du poids de la composition de cholécalciférol, de vitamine D3; de 50 à 80% du poids de la composition d'au moins un émulsifiant, de 2 à 6% du poids de la composition d'au moins un agent désintégrant, de 2 à 10% du poids de la composition d'au moins un agent diluant, de 0,1 à 2% en poids de la composition d'au moins un liant, du 0,2 à 0,5% du poids de la composition d'un premier recouvrement entérique, de 3 à 5% du poids de la composition d'un deuxième recouvrement entérique, de 0,25 à 5% du poids de la composition d'au moins un agent lubrifiant et une quantité suffisante d'un ou plusieurs solvants ou un mélange de solvants comme milieu de dissolution.

3. Composition selon la revendication 1, dans laquelle le diluant est choisi parmi le groupe comprenant de la cellulose PH 102, de la cellulose microcristalline PH 101, du Ludipress, du Prosolv 90; l'agent désintégrant est choisi parmi le groupe consistant en croscarmellose sodique, crospovidone et du glycolate d'amidon sodique; le liant est choisi parmi le groupe consistant en polyvinylpyrrolidone K 30, povidone PK 90, copovidone, hydroxypropylméthylcellulose, éthylcellulose; l'agent émulsifiant est choisi parmi le groupe consistant en Phosal 50 PG, phosphatidylcholine en gélatine de soja, propylène glycol, des mono-et diglycérides de tournesol et du palmitate d'ascorbyl; la composition comprenant un recouvrement entérique est choisi à partir de SN entérique et de l'alginate de sodium, de l'Acryleze™, du copolymère d'acide méthacrylique, du Sureteric®, des polyméthacrylates, du triéthylcitrate, de la triacétine et du polyéthylène glycol; et les solvants sont choisis à partir d'eau purifiée, de l'éthanol, du propanol et leur mélanges.

4. Composition selon la revendication 1, qui est **caractérisée en ce que** la composition de granulés du risédronate comprend du risédronate de sodium et un agent désintégrant choisi parmi le groupe consistant en croscarmellose sodique, cellulose microcristalline et povidone K30.

5. Composition selon la revendication 1, qui est **caractérisée en ce que** la protection de cholécalciférol consiste en un émulsifiant choisi parmi le groupe comprenant du Phosal, du la phosphatidylcholine en lécithine de soja, du propylène glycol, des mono- et diglycérides de tournesol et du palmitate d'ascorbyl et de l'éthanol comme solvant.

6. Composition selon la revendication 1, qui est **caractérisée en ce que** la solution de cholécalciférol est pulvérisée sur le risédronate de sodium, et postérieurement recouverte d'une suspension de recouvrement de SN entérique.

7. Composition selon la revendication 1, qui est **caractérisée en ce que** le comprimé comprend aussi un ou plusieurs excipients choisis parmi la croscarmellose sodique, la cellulose microcristalline, la povidone K30, le Phosal et un agent SN entérique et un lubrifiant, comme stéarate de magnésium.

8. Composition selon la revendication 1, qui est **caractérisée en ce que** le recouvrement consiste en un copolymère d'acide méthacrylique.

9. Procédé de préparation de la composition selon la revendication 1, **caractérisée en ce qu'**elle comprend les étapes suivantes
a) préparer la composition de granulés
b) préparer la protection de vitamine D3
c) recouvrir le granulé d'une couche entérique
d) former le noyau du comprimé; et
e) recouvrir le noyau de comprimé et la vitamine D3 protégé avec une couche entérique.

10. Procédé selon la revendication 9, qui est **caractérisé en ce que** la préparation de la composition de granulés consiste en les étapes suivantes:
a) peser, mélanger et granuler risédronate de sodium, croscarmellose sodique, cellulose microcristalline PH 102 et polyvinylpyrrolidone K 30 avec de l'eau purifié.
b) tamiser en utilisant une maille numéro 14 et sécher dans un four jusqu'à atteindre au moins 3,0% d'humidité.

11. Procédé selon la revendication 9, qui est **caractérisé en ce que** la préparation de la protection de vitamine D3 consiste en les étapes suivantes:
a) dissoudre la vitamine D3 dans du Phosal 50 PG et ensuite diluer avec de l'alcool éthylique, mélanger jusqu'à former une solution jaune claire.
b) introduire les granules secs de risédronate dans une cuvette de lit fluide, et le perméat avec la solution de pulvérisation de vitamine D3 à une température entre 25º à 30ºC.

12. Procédé selon la revendication 9, qui est **caractérisé en ce que** la préparation du recouvrement entérique de granulés consiste en les étapes suivantes:
a) préparer une suspension de SN Entérique dans de l'eau purifiée à 10% de solides, agiter jusqu'à son incorporation complète et tamiser en utilisant une maille numéro 50.
b) atomiser la suspension précitée dans les granulés à une température de 25º à 35ºC, sécher les granulés dans un lit fluide et tamiser en utilisant une maille numéro 18.

13. Procédé selon la revendication 9, qui est **caractérisé en ce que** la formation de noyau de granule consiste en les étapes suivantes:
a) tamiser en utilisant une maille numéro 20: cellulose microcristalline PH 102, croscarmellose sodique et mélanger avec les granulés de l'étape 6 pendant 5 minutes.
b) lubrifier avec du stéarate de magnésium, tamisé préalablement à travers une maille numéro 30 pendant 3 minutes.
c) comprimer dans une presse hexagonale de 4 mm à 200 mg ± 5% de poids.

14. Procédé selon la revendication 9, qui est **caractérisé en ce que** la préparation du recouvrement entérique pour le noyau de comprimé et la vitamine D3 protégée consiste en les étapes suivantes:
a) préparer une suspension de recouvrement avec de l'Acryl-eze™ et du triéthylcitrate dans de l'eau purifiée à 20% de solides.
b) recouvrir les comprimés avec la suspension d'Acryl-eze™ à une température de 30º à 35ºC dans un tambour perforé.
c) préparer la suspension de recouvrement avec de l'Opadry® FX dans de l'eau purifiée à 6.5% de solides.
d) recouvrir les comprimés avec la suspension d'Opadry® FX à une température de 38º à 43ºC dans un tambour perforé.

15. Composition selon la revendication 1, qui est **caractérisée en ce que** le cholécalciférol a une stabilité supérieure à 93% après 4 semaines à 30ºC/65% d'humidité relative, 40ºC/75% d'humidité relative, et 40ºC/75% d'humidité relative cyclique et refroidissement de l'humidité relative (2ºC - 8ºC).

16. Composition selon la revendication 1, qui est **caractérisé en ce que** la dissolution de risédronate n'était pas supérieure à 10,0 à pH acide et est de 100% à pH physiologique à 10 minutes.
